(19) **Europäisches Patentamt / European Patent Office / Office européen des brevets**

(11) **EP 4 772 616 A1**

(12) **EUROPEAN PATENT APPLICATION**
published in accordance with Art. 153(4) EPC

(43) Date of publication:
**08.07.2026 Bulletin 2026/28**

(21) Application number: **24859798.1**

(22) Date of filing: **28.08.2024**

(51) International Patent Classification (IPC):
*C12N 7/01* (2006.01)        *C12N 1/00* (2006.01)
*C12N 5/10* (2006.01)        *C12N 5/073* (2010.01)
*C12N 15/63* (2006.01)        *C12N 15/87* (2006.01)
*C12N 15/88* (2006.01)        *C12N 15/864* (2006.01)

(52) Cooperative Patent Classification (CPC):
**C12N 1/00; C12N 5/06; C12N 5/10; C12N 7/00;
C12N 15/63; C12N 15/864; C12N 15/87;
C12N 15/88**

(86) International application number:
**PCT/JP2024/030577**

(87) International publication number:
**WO 2025/047766 (06.03.2025 Gazette 2025/10)**

(84) Designated Contracting States:
**AL AT BE BG CH CY CZ DE DK EE ES FI FR GB
GR HR HU IE IS IT LI LT LU MC ME MK MT NL
NO PL PT RO RS SE SI SK SM TR**
Designated Extension States:
**BA**
Designated Validation States:
**GE KH MA MD TN**

(30) Priority: **29.08.2023 JP 2023138705
28.06.2024 JP 2024104516**

(71) Applicant: **FUJIFILM Corporation
Tokyo 106-8620 (JP)**

(72) Inventors:
• **NISHINO Masafumi
  Ashigarakami-gun, Kanagawa 258-8577 (JP)**
• **MURAGUCHI Taichi
  Ashigarakami-gun, Kanagawa 258-8577 (JP)**

• **NAGAI Yoichi
  Ashigarakami-gun, Kanagawa 258-8577 (JP)**
• **EDO Michiko
  Ashigarakami-gun, Kanagawa 258-8577 (JP)**
• **MORI Yusuke
  Ashigarakami-gun, Kanagawa 258-8577 (JP)**
• **ISHII Satomu
  Ashigarakami-gun, Kanagawa 258-8577 (JP)**

(74) Representative: **Hoffmann Eitle
Patent- und Rechtsanwälte PartmbB
Arabellastraße 30
81925 München (DE)**

Remarks:
The complete document including Reference Table(s) and the Sequence Listing(s) can be downloaded from the EPO website

(54) **VIRUS PRODUCTION METHOD AND METHOD FOR INTRODUCING NUCLEIC ACID INTO CELL**

(57)    An object of the present invention is to provide a method for improving a proportion of capsids containing a gene of interest GOI in a method for producing a virus, and to provide a method for improving a proportion of capsids containing a gene of interest GOI in a method for introducing a nucleic acid into cells, the method including a nucleic acid introduction step of adding a nucleic acid to a suspension containing cells. According to the present invention, there is provided a method for producing a virus, the method including a nucleic acid introduction step of adding a nucleic acid to a suspension containing cells having a density of $10 \times 10^6$ cells/mL or more, and a culture step of culturing the cells into which the nucleic acid has been introduced, in which the nucleic acid is added in a case where a population cell doubling time of the suspension satisfies 35.4 hours or less.

# FIG. 1

## Description

## Technical Field

The present invention relates to a method for producing a virus, the method including a nucleic acid introduction step of adding a nucleic acid to a suspension containing cells, and a culture step of culturing the cells into which the nucleic acid has been introduced. The present invention further relates to a method for introducing a nucleic acid into cells, the method including a nucleic acid introduction step of adding a nucleic acid to a suspension containing cells.

## Background Art

A gene therapy method has been developed in which a recombinant adeno-associated virus (AAV) vector into which a therapeutic gene has been incorporated is administered to a patient for the purpose of treatment of an intractable disease such as a genetic disease, cancer, or the like. As a method for producing an AAV vector, a method has been known in which a complex is formed between a plasmid DNA having a negative charge and polyethyleneimine (PEI) as a cationic polymer to neutralize the charge of the plasmid DNA, a plasmid DNA encoding proteins necessary for AAV production is introduced into cells by endocytosis, and AAV is produced in the cells.

Patent Document 1 describes a method for transfecting cells with at least one nucleic acid sequence, the method including: (a) a step of bringing the cells into contact with at least one nucleic acid that is formulated in a solution containing polyethyleneimine (PEI); (b) a step of incubating or culturing the cells together with a nucleic acid and a polyethyleneimine (PEI) solution; (c) a step of adding an enhancing agent to produce a mixture at the time point of step (a) or immediately after step (a), or within 3 hours from step (a); and (d) a step of incubating the mixture of step (c) to transfect the cells with the nucleic acid sequence.

Patent Document 2 describes a method for producing an adeno-associated virus (AAV), the method including: (a) a step of preparing a stable AAV producer host cell line; and (b) a step of culturing the cells by perfusion culture during the AAV production step, in which the perfusion culture includes continuous replacement of spent culture medium with fresh culture medium and the continuous replacement of the spent culture medium with the fresh culture medium follows the induction of AAV production.

## Prior Art Documents

## Patent Documents

Patent Document 1: JP2020-524498A

Patent Document 2: JP2023-518919A

## Summary of Invention

## Object to be solved by the invention

To achieve high productivity of a virus (for example, AAV), it is important to use cells having high productivity and to increase the cell density. However, it has been found that simply increasing the cell density does not increase the number of capsids containing a gene of interest (GOI) (for example, a gene for treatment or prevention). An object to be achieved by the present invention is to provide a method for improving a proportion of capsids containing a GOI in a method for producing a virus. Another object to be achieved by the present invention is to provide a method for improving a proportion of capsids containing a GOI in a method for introducing a nucleic acid into cells, the method including a nucleic acid introduction step of adding a nucleic acid to a suspension containing cells.

## Means for solving the object

As a result of intensive studies to achieve the above-described object, the present inventors have focused on a process in which the GOI is generated in the cells, and have hypothesized that in cells with proliferative activity approaching saturation, the capsids containing the GOI cannot be generated due to a decrease in the replication of the GOI. The present inventors have found, based on the above hypothesis, that the high productivity of a virus (AAV) can be achieved by transfecting the cells in a state in which cell division is activated. The present invention has been completed based on the above findings.

According to an aspect of the present invention, the following invention is provided.

<1> A method for producing a virus, the method comprising: a nucleic acid introduction step of adding a nucleic acid to a suspension containing cells having a density of $10 \times 10^6$ cells/mL or more; and a culture step of culturing the cells into which the nucleic acid has been introduced, in which the nucleic acid is added in a case where a population cell doubling time of the suspension satisfies 35.4 hours or less.

<2> The method according to <1>, in which a time at which the population cell doubling time of the suspension satisfies 35.4 hours or less is a time at which a cell density A at a time point A and a cell density B at a time point B which is 24 hours before the time point A satisfy the cell density A/the cell density B $\geq 1.6$.

<3> The method for producing a virus according to <1> or <2>, further comprising: performing perfusion culture of the cells before the nucleic acid introduction step.

<4> The method for producing a virus according to <3>, in which a perfusion ratio at a time point 24 hours before a start of the nucleic acid introduction step is 1 or more.

<5> The method for producing a virus according to any one of <1> to <4>, further comprising: a preculture step of culturing the suspension containing the cells having a density of $10 \times 10^6$ cells/mL or more before the nucleic acid introduction step.

<6> The method for producing a virus according to any one of <1> to <5>, in which, in the nucleic acid introduction step, the nucleic acid is introduced into the cells using a gene introduction reagent or by electroporation.

<7> The method for producing a virus according to any one of <1> to <6>, in which a concentration of the nucleic acid in the suspension is 3 μg/mL or more.

<8> The method for producing a virus according to <6>, in which the gene introduction reagent is a cationic polymer.

<9> The method for producing a virus according to any one of <6> to <8>, in which a mass ratio of the nucleic acid to the gene introduction reagent is 1:1 to 1:3.

<10> The method for producing a virus according to any one of <1> to <9>, in which the virus is an adeno-associated virus.

<11> The method for producing a virus according to <10>, in which the nucleic acid includes one or more selected from an adeno-associated virus gene and a virus helper gene.

<12> The method for producing a virus according to <10> or <11>, in which the nucleic acid includes at least one or more virus helper genes.

<13> The method for producing a virus according to any one of <10> to <12>, in which the nucleic acid contains four or more genes selected from the group consisting of a gene for treatment or prevention, a Rep gene, a Cap gene, an E2 gene, an E4 gene, and a VA-RNA1 gene.

<14> The method for producing a virus according to any one of <1> to <13>, in which the cells are animal cells.

<15> The method for producing a virus according to any one of <1> to <14>, in which the cells are HEK cells.

<16> The method for producing a virus according to any one of <1> to <15>, in which the nucleic acid is introduced into the cells using one or more plasmids.

<17> The method for producing a virus according to any one of <1> to <16>, further comprising: a preculture step of culturing the suspension containing the cells having a density of $10 \times 10^6$ cells/mL or more before the nucleic acid introduction step, in which, in the culture step, perfusion culture is not performed for 4 to 24 hours immediately after the nucleic acid introduction step, and subsequently perfusion culture is performed.

<18> The method for producing a virus according to any one of <1> to <16>, further comprising: a preculture step of culturing the suspension containing the cells having a density of $10 \times 10^6$ cells/mL or more, in which, in a case where a population cell doubling time of the suspension satisfies 35.4 hours or less, a part of a culture product of pre-culture is collected, a nucleic acid is added to a part of the collected culture product, and the cells into which the nucleic acid has been introduced are cultured to produce a virus, and culture of an uncollected remaining part of the culture product of the pre-culture is continued, and subsequently, in a case where a population cell doubling time of the suspension satisfies 35.4 hours or less, a part of the culture product is collected and viral production by nucleic acid introduction and cell culture is performed.

<19> A method for introducing a nucleic acid into cells, the method comprising: a nucleic acid introduction step of adding a nucleic acid to a suspension containing cells having a density of $10 \times 10^6$ cells/mL or more, in which the nucleic acid is added in a case where a population cell doubling time of the suspension satisfies 35.4 hours or less.

**Effect of the invention**

According to the present invention, in a method for producing a virus, the method including a nucleic acid introduction step of adding a nucleic acid to a suspension containing cells, and a culture step of culturing the cells into which the nucleic acid has been introduced, a proportion of capsids containing a GOI can be improved.

## BRIEF DESCRIPTION OF THE DRAWINGS

[FIG. 1] FIG. 1 shows a result of measuring a change in cell density during a culture period.
[FIG. 2] FIG. 2 shows a measurement result of a genome titer of AAV5.
[FIG. 3] FIG. 3 shows a measurement result of a copy number of a gene of interest.
[FIG. 4] FIG. 4 shows a measurement result of a Full ratio.
[FIG. 5] FIG. 5 is a top view of an electroporation device 100.
[FIG. 6] FIG. 6 is a cross-sectional view of the electroporation device 100.

## Embodiments for carrying out the invention

Hereinafter, an example of the embodiment of the present disclosure will be described. However, the present disclosure is not limited to the following embodiments, and can be implemented with appropriate changes within the scope of the object of the present disclosure. In the present specification, the numerical range indicated by using "to" means a range including numerical values described before and after "to" as a minimum value and a maximum value, respectively.

The present invention is a method for producing a virus, the method including a nucleic acid introduction step of adding a nucleic acid to a suspension containing cells having a density of $10 \times 10^6$ cells/mL or more, and a culture step of culturing the cells into which the nucleic acid has been introduced, in which the nucleic acid is added in a case where a population cell doubling time of the suspension satisfies 35.4 hours or less. By satisfying the above, the cells are transfected in a highly activated state of cell division, whereby the high productivity of the virus can be achieved.

The population cell doubling time (T) can be calculated by the following calculation expression.

$$T = n \times LN(2)/LN\{(d_A \times V_A)/(d_B \times V_B)\}$$

$d_A$: cell density (cells/mL) at the time point A
$V_A$: volume (mL) of suspension containing cells at the time point A
$d_B$: cell density (cells/mL) at the time point B (n hours before the time point A)
$V_B$: volume (mL) of suspension containing cells at the time point B
n: time from the time point B to the time point A

In a case where cell bleeding (CB) is included between the time point B and the time point A, the population cell doubling time (T) can be calculated by the following calculation expression.

$$T = n \times LN(2)/LN\{(d_A \times V_A + \int d_{CB} \times V_{CB} dt)/(d_B \times V_B)\}$$

$d_A$: cell density (cells/mL) at the time point A
$V_A$: volume (mL) of suspension containing cells at the time point A
$d_B$: cell density (cells/mL) at the time point B (n hours before the time point A)
$V_B$: volume (mL) of suspension containing cells at the time point B
$d_{CB}$: cell density of suspension (cells/mL) containing cells at each time point of cell bleeding
$V_{CB}$: volume (mL) of suspension containing cells to be cell-bleed at each time point of cell bleeding
n: time from the time point B to the time point A

The population cell doubling time of the suspension may be 35.4 hours or less, but is preferably 31.5 hours or less, more preferably 28.0 hours or less, and particularly preferably 24.0 hours or less.

The cell used in the present invention may be any one of an isolated cell, a cell included in an isolated living body-derived tissue, or a cultured cell. In a case where the cell is an isolated cell or a cultured cell, the cell may be any of an adherent cell or a floating cell.

As the cell, an animal cell is preferable. The animal cell is not particularly limited, but is preferably a mammalian cell or an insect cell, and more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, baby hamster kidney cells (BHK cells), VERO, SP2/0, YB2/0, Y0, C127, L cells, COS (for example, COS1 and COS7), QC1-3, human embryonic kidney cells (HEK cells) cells (for example, HEK293 and the like), PER. C6, HeLa, EB1, EB2, EB3, and oncolytic or hybridoma cell lines. The cell is preferably a HEK cell or a CHO cell, and more preferably a HEK293 cell. The cells may be either adherent cells or suspension cells, but suspension cells are preferable to perform high-density large-scale culture in a reactor.

As a cell line having high cell proliferative activity and high AAV production ability per cell, for example, a suspension HEK293 cell line Viral Production Cells 2.0 (VPCs 2.0) manufactured by Thermo Fisher Scientific, Inc. can be used.

The method for producing a virus according to the embodiment of the present invention may include pre-culturing the cells before the nucleic acid introduction step. Preferably, in the present invention, a preculture step of culturing the suspension containing cells having a density of $10 \times 10^6$ cells/mL or more may be included before the nucleic acid introduction step.

The method of preculture of cells is not particularly limited, and may be any of batch culture, fed-batch culture, perfusion culture, shaking culture, stirring culture, standing culture, or adhesion culture. From the viewpoints of large-scale treatment and high concentration, perfusion culture is preferable. In the perfusion culture, the removal of waste components by a filter and the supply of a fresh culture medium make it possible to perform proliferation at a high concentration by maintaining the quality (component concentration) of the suspension under conditions suitable for cell proliferation.

The temperature of the preculture of the cells is preferably 30°C to 40°C, more preferably 32°C to 37°C, and particularly preferably 36°C to 37°C. The $CO_2$ concentration is 2% to 25%.

It is preferable to culture such that the cell density of the preculture is $10 \times 10^6$ cells/mL or more and $200 \times 10^6$ cells/mL or less. The cell density is more preferably $15 \times 10^6$ cells/mL or more and $100 \times 10^6$ cells/mL or less, and more preferably $20 \times 10^6$ cells/mL or more and $80 \times 10^6$ cells/mL or less.

The culture time of the preculture is preferably 12 hours or more.

In a case of performing perfusion culture of the cells before the nucleic acid introduction step, from the viewpoint of increasing the proliferative activity of the cells, a perfusion ratio at a time point 24 hours before a start of the nucleic acid introduction step is preferably 0.5 or more and more preferably 1 or more. The perfusion ratio is still more preferably 1 to 5. The perfusion ratio may be 2 to 5 or 3 to 5. The perfusion ratio is a ratio of a volume of the cell culture solution replaced in one day. The perfusion ratio of 1 means that the entire volume (1-fold volume) is replaced in one day. The perfusion ratio of 3 means that the 3-fold volume of culture solution is replaced in one day.

In a case of perfusion culture, either a dynamic mode or a continuous mode may be used. In the dynamic method, cells are seeded at a low concentration, proliferative culture is performed, the total volume of the cell solution is collected after reaching a high concentration, and gene introduction is performed. In the continuous mode, cells are seeded at a low concentration, proliferated culture is performed, a certain volume of cells are extracted (cell bleeding) after reaching a certain concentration, and gene introduction is performed. Fresh culture medium in the same volume as the extraction volume is supplemented on the reactor side to maintain the constant cell concentration in a reactor. The cell bleeding and the supply of the culture medium may be continuous or intermittent (for example, once a day at 10% to 50% or the like). The concentration is recovered by cell proliferation until the next cell bleeding. The above operation can be repeated, for example, for 1 day to 3 months. The perfusion ratio is not particularly limited; however, it is generally 0.3 vvd to 5.0 vvd preferably 0.5 vvd to 4.0 vvd. The vvd regarding the perfusion ratio means "volume of withdrawn culture solution/volume of culture solution in culture container/day"

Devices and culture methods for perfusion culture are known in the related art, and are described in WO2018/159847, WO2019/049843A, WO2019/181234A, WO2019/239780A, WO2020/003833A, WO2020/162125A, WO2021/187008A, WO2022/196710A, and WO2023/054556A, the contents of which are incorporated herein by reference. In addition, perfusion culture which will described later can also be used in the preculture. For example, the description of the "membrane separation treatment step of passing a cell suspension extracted from a culture tank through a separation membrane to separate the cell suspension into a cell-containing liquid and a permeated liquid" can be used in the preculture.

The method for producing a virus according to the embodiment of the present invention includes a nucleic acid introduction step of adding a nucleic acid to a suspension containing cells having a density of $10 \times 10^6$ cells/mL or more.

A density of the cells in the suspension to which the nucleic acid is to be added may be $10 \times 10^6$ cells/mL or more, but is preferably $12 \times 10^6$ cells/mL or more, more preferably $15 \times 10^6$ cells/mL or more, still more preferably $18 \times 10^6$ cells/mL or more, and particularly preferably $20 \times 10^6$ cells/mL or more.

The upper limit of the cell density is $100 \times 10^6$ cells/mL. In a case where the upper limit is exceeded, it is difficult for the population cell doubling time of the suspension to satisfy 35.4 hours or less, and the replication of the gene for treatment is decreased.

The cell density can be determined using Vi-cell (trademark) XR (manufactured by Beckman Coulter, Inc.).

A time at which the population cell doubling time of the suspension satisfies 35.4 hours or less may be a time at which a cell density A at a time point A and a cell density B at a time point B which is 24 hours before the time point A satisfy the cell density A/the cell density B ≥ 1.6.

The addition of the nucleic acid may be performed at any time as long as the cell growth phase in which the increase rate of the cell density in the above 24 hours is 1.6 or more. The increase rate of the cell density over the above 24 hours is more preferably 1.7 or more, more preferably 1.8 or more, and still more preferably 2 or more.

The cell proliferation has a lag phase, a logarithmic growth phase, a stationary phase, and a death phase. The present invention is intended to perform transfection in a predetermined period of the logarithmic growth phase. It is noted that the increase rate gradually decreases as a certain time elapses. In a case where the present inventors have attempted transfection at the high cell density, the transfection is performed in a period in which the increase rate of the cell density is low, and the amount of the capsids produced containing the GOI is decreased. In the present invention, the transfection is performed in the period in which the cell increase rate is high even at the high cell density, whereby the amount of the capsids produced containing the GOI can be improved.

In the cell culture, the cell density is measured for a certain time every day. For example, the cell density is measured once a day at 9:00 a.m.

After the start of the culture, the cell density A is measured at a certain 9:00 a.m. (time point A), in a case where it is confirmed that the cell density has reached $10 \times 10^6$ cells/mL or more from the start of the cell culture, and that the cell density B measured at 9:00 a.m. (time point B) on the previous day and the cell density A satisfy the above expression, the nucleic acid is added.

It is preferable that the nucleic acid is added immediately after the above 9:00 a.m. in a case where the above-described conditions are satisfied. It is not particularly critical in a case where the time is slightly delayed, but it is preferably within 3 hours, more preferably within 2 hours, and still more preferably within 1 hour from the time point A.

In addition, in a case where it is known that the above-described conditions are satisfied as a predetermined time has elapsed from the start of the culture, the nucleic acid may be added after the predetermined time has elapsed from the start of the culture without measuring the cell density.

In the nucleic acid introduction step, the nucleic acid is preferably introduced into the cells using a gene introduction reagent or by electroporation.

The nucleic acid refers to a chain-like polynucleotide in which a nucleotide consisting of a purine or pyrimidine base, a sugar, and a phosphate is a basic unit, and the phosphate forms a diester bond between the 3' and 5' carbon atoms of the sugar between each of the nucleotides to polymerize. Due to the difference in sugar, nucleic acids are roughly classified into deoxyribonucleic acid (DNA) having a sugar moiety of deoxyribose and ribonucleic acid (RNA) having a sugar moiety of ribose. The nucleic acid may be any one of a gene, DNA (linear DNA or circular DNA), RNA, an oligonucleotide, or a polynucleotide.

In the present specification, the nucleic acid is used interchangeably with a gene, DNA, RNA, an oligonucleotide, and a polynucleotide.

An example of the nucleic acid in the present invention is a plasmid.

The gene introduction reagent is preferably a cationic polymer. The cationic polymer is not particularly limited, and for example, one type selected from the group consisting of chitosan, poly-L-lysine (pLL), polyamine (PA), polyalkyleneimine (PAI), polyethyleneimine (PEI), poly[$\alpha$-(4-amino butyl)-L-glycolic acid], polyamidoamine, poly(2-dimethylamino)ethyl methacrylate, polyhistidine, polyarginine, poly(4-vinylpyridine), poly(vinylamine), and poly(4-vinyl-N-alkylpyridinium halide), or a combination thereof can be used.

As the cationic polymer, polyamine is preferable, and polyethyleneimine (hereinafter, also referred to as PEI) is particularly preferable. As the cationic polymer, it is preferable to use a non-lipid cationic polymer.

The PEI may be linear PEI or branched PEI, but is preferably linear PEI.

The molecular weight of PEI is preferably 10,000 to 800,000 Da, more preferably 10,000 to 50,000 Da, still more preferably 10,000 to 27,000 Da, and particularly preferably about 25,000 Da, in consideration of the transfection efficiency. As an example, linear PEI having a molecular weight of about 25,000 Da can be used. PEI is commercially available and can be easily obtained. For example, PEIpro manufactured by Polyplus can be preferably used.

In electroporation (EP), the perforation site is formed in cells by an electric pulse application, and a plasmid DNA that is negatively charged and repelled by the cell is brought close to and penetrated into the cells (the perforation site) by an electrophoresis action due to the electric pulse application, thereby realizing intracellular gene introduction. Therefore, unlike the PEI method, since the gene introduction in the EP method is completed at the time of pulse application, it is not necessary to control stirring and culture for a long time as in the PEI method, and the cell concentration can be temporarily changed freely only in the EP step as long as uniform dispersibility of the cells and the plasmid DNA can be maintained. In addition, since the introduction is based on the principle of electrophoresis, it is not necessary to individually optimize the concentration of added plasmid according to cell concentration or the state of the culture medium, and the same plasmid concentration condition can be applied in a wide range of cell concentrations. Therefore, from the viewpoint of improving the efficiency of using the plasmid and the viewpoint of reducing the amount of the treatment, it is preferable to set the cell concentration at the time of EP to be high.

The cell concentration may be increased as compared with the cell concentration at the time of the preculture, by cell concentration using centrifugation, continuous centrifugation, sonic agglomeration, acoustic electrophoresis, filtration (TFF, ATF), or the like. It is preferable that the concentration is performed to a cell concentration of $80 \times 10^6$ cells/mL or more. The cell concentration is more preferably $100 \times 10^6$ cells/mL or more and $120 \times 10^6$ cells/mL or more. In addition, in the same step, the culture medium of the suspension may be exchanged with a fresh culture medium, an electroporation dedicated buffer, or a culture medium optimized for subsequent culture.

As the conditions of the buffer at the time of EP, conditions suitable for typical cell culture (pH, pH buffering capacity, nutrients, salt concentration, and the like) can be used.

In the nucleic acid introduction step, it is preferable that the cells are in a suspension, and the conductivity of the suspension is 5 to 20 mS/cm. Provided that since it is possible to take measures to reduce heat generation under the conditions of electroporation, it is not essential that the conductivity in the suspension is 5 to 20 mS/cm.

In the electroporation, a membrane repair agent may be added, or a nutritional component may be added.

There are many similarities in the functions and components between the electroporation buffer and the culture medium for cell culture, and in some cases, the cell viability after EP, and the efficiency of gene introduction and expression may be increased by using a dedicated culture medium optimized for each cell, rather than a general-purpose EP buffer. In the HEK293 cell × EP plasmid introduction, it is preferable to use a Balan-CD-HEK293 medium.

In addition, in a case where the concentration of waste products and nutrients in the culture medium can be maintained at a certain quality by perfusion culture or the like in the preculture, the buffer exchange may be omitted, and the preculture suspension may be applied to the EP as it is. By omitting the concentration and culture medium exchange step, it is possible to achieve step simplification, reduction of contamination risk, improvement of process stabilization and reproducibility, and reduction of cost.

The type of power supply does not matter as long as the required conditions of the applied voltage (required electric field E × gap) and the power capacity (pulse current, pulse duration, pulse interval) are satisfied. The function generator is several tens of V and several tens of μm, and the function generator + power amplifier is several hundreds of V and 2 mm gap. The dedicated pulse power supply (DC power supply + capacitor + power switch circuit) is ~ several kV and ~ 1 cm gap.

The liquid feeding pump and the liquid feeding method may be any of a syringe pump, a tube pump, a magnet pump, a diaphragm pump, or the like, or may be pressurized air feeding.

The electroporation may be any of batch electroporation (single-use cuvette (several tens of μL to about 1 mL, pipetting work)), continuous batch electroporation (several mL to several hundreds of mL), or continuous electroporation (several mL to several tens of L). In a case where the treatment amount (volume of culture solution) is large, continuous electroporation is preferable. In the case of continuous electroporation, a liquid feeding method with reduced pulsation is preferable.

The device for performing electroporation is not particularly limited. The device is known in the related art, and is described in WO2022/224803A, WO2023/157673A, and WO2023/223931A, the contents of which are incorporated herein by reference.

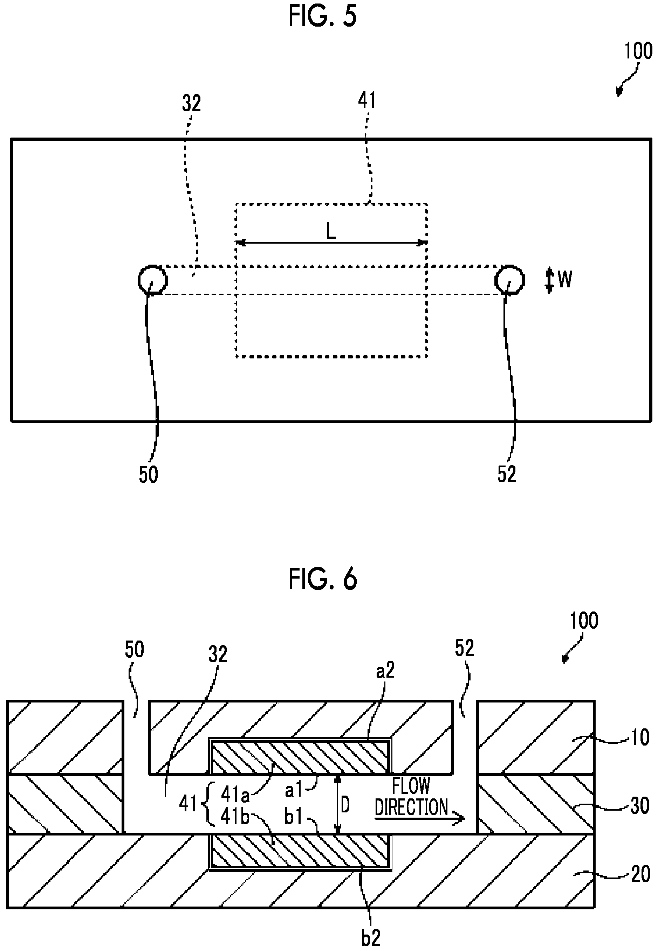

As an example of a device for performing continuous electroporation, the electroporation device shown in FIGS. 5 and 6 can be used. FIG. 5 is a top view of the electroporation device 100, and FIG. 6 is a cross-sectional view of the electroporation device 100.

In the electroporation device 100, the upper electrode 41a held by the upper electrode holding plate 10 and the lower electrode 41b held by the lower electrode holding plate 20 are installed to face each other to constitute an electrode pair 41. A power supply (not shown) is connected to the upper electrode 41a and the lower electrode 41b. D represents an inter-electrode distance (gap). In FIG. 5, L represents an electrode length, and W represents an electrode width (flow channel width). In FIG. 6, a1 represents a front surface portion of the upper electrode, a2 represents a rear surface portion of the upper electrode, b1 represents a front surface portion of the lower electrode, and b2 represents a rear surface portion of the lower electrode. A flow channel plate 30 is provided between the upper electrode holding plate 10 and the lower electrode holding plate 20. The fluid introduced from the inflow port 50 is discharged from the outflow port 52 through the opening portion (flow channel) 32.

It is preferable that the supply reservoir, the liquid feeding tube, the electroporation device, and the collection reservoir are aseptically connected to each other. That is, in the present invention, it is preferable that the liquid feeding is performed aseptically in the nucleic acid introduction step and the culture step. The preculture step (including concentration, culture medium exchange, and mixing system) and the subsequent culture step (culture step of culturing the cells into which the nucleic acid has been introduced) may be continuously connected or may be separated.

A concentration of the nucleic acid in the suspension to which the nucleic acid has been added is preferably 3 μg/mL or more, more preferably 4 μg/mL or more, and still more preferably 5 μg/mL or more.

In a case of using the gene introduction reagent, a mass ratio of the nucleic acid to the gene introduction reagent is preferably 1:1 to 1:3 and more preferably 1:2.

As the nucleic acid, a nucleic acid encoding a virus can be used, and preferably a exogenous recombinant nucleic acid encoding a virus can be used.

In the present invention, a virus is produced. The virus to be produced may be used, for example, as an active pharmaceutical ingredient.

The virus means a virus that is produced by introducing a nucleic acid into a cell. Examples of the virus include a non-enveloped virus. More specific examples thereof include an adeno-associated virus, an adenovirus, a lentivirus, a baculovirus, and a retrovirus, and among these, an adeno-associated virus is preferable.

The non-enveloped virus is known in the related art, and is described in WO2015/005430A, the contents of which are incorporated herein by reference.

Adeno-associated virus (AAV) refers to a small, incompletely replicative, non-enveloped virus containing single-stranded DNA having about 4,700 bases, from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue tropisms. It is said that AAV1 has high gene introduction efficiency into muscle, liver, airway, central nervous system, and the like, AAV5 has high gene introduction efficiency into central nervous system, liver, retina, and the like, and AAV6 has high gene introduction efficiency into heart, muscle, liver, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

AAV is a non-enveloped virus that proliferates in the presence of helper viruses such as adenovirus and herpesvirus. In the preparation of AAV to be used for gene therapy or nucleic acid introduction, classically, AAV replication has been performed by co-infecting host cells with an adenovirus. In addition, a gene responsible for the helper action of adenovirus has been clarified, and a plasmid bearing this gene has also been used. For example, a plasmid containing a Rep gene and a Cap gene, an adenovirus helper plasmid, and a plasmid containing a gene for treatment or prevention are simultaneously transfected into cells, and can thus be packaged as recombinant AAV (rAAV).

The nucleic acid used in the present invention preferably includes one or more selected from an adeno-associated virus gene and a virus helper gene. The nucleic acid preferably includes at least one or more virus helper genes.

The Rep gene and the Cap gene encode proteins involved in the replication and packaging of the virion. In the wild type, the Rep gene is expressed from the P5 promoter and the p19 promoter. The Cap region expresses VP1, VP2, and VP3. Examples of the promoter that is naturally carried by the Cap gene can include a p40 promoter.

The adeno-associated virus gene (such as a Rep gene and a Cap gene) may be a wild-type gene, but a gene in which modifications such as substitution, deletion, insertion, or addition of a base have been made to the wild-type gene may be used as long as it exhibits an inherent function.

In a case where the Rep gene and the Cap gene are introduced into a cell, a vector containing the Rep gene and the Cap gene can be introduced into the cell. The Rep gene means a region of the AAV genome encoding a virus replication protein that is known to those skilled in the art and that is collectively required for replication of a virus genome, or its functional homolog, for example, a human herpesvirus 6 (HHV-6) Rep gene, or the like (mediating AAV-2 DNA replication is known). The Cap gene means a region in the AAV genome encoding a capsid protein of a virus known to those skilled in the art.

As the vector containing the Rep gene and the Cap gene, for example, a plasmid, a nucleic acid sequence derived from a virus, or an artificially designed nucleic acid can be used, and a plasmid is preferable.

In a case where modifications such as substitution, deletion, insertion, or addition of a base are made to the wild-type adeno-associated virus gene (such as a Rep gene and a Cap gene), the number of the modified bases is preferably 1 to 20, more preferably 1 to 10, and still more preferably 1 to 3. The modified base sequence of the adeno-associated virus gene exhibits preferably 85% or more sequence identity, more preferably 90% or more sequence identity, still more preferably 95% or more sequence identity, and even still more preferably 98% or more sequence identity with the base sequence of the wild-type adeno-associated virus gene.

In a case where the Rep gene and the Cap gene are introduced into a cell, a vector containing the Rep gene and the Cap gene can be introduced into the cell. The arrangement of the Rep gene and the Cap gene in a vector is not particularly limited, the Rep gene may be located upstream of the Cap gene or downstream of the Cap gene, and the Rep gene is located preferably upstream of the Cap gene.

The Rep gene means a region of the AAV genome encoding a virus replication protein that is known to those skilled in the art and that is collectively required for replication of a virus genome, or its functional homolog, for example, a human herpesvirus 6 (HHV-6) Rep gene, or the like (mediating AAV-2 DNA replication is known). Accordingly, the coding region of the Rep gene includes at least a gene encoding REP78 and REP68 (long form of REP protein) and REP52 and REP40 (short form of REP protein) of AAV, or a functional homologue thereof.

The Cap gene means a region in the AAV genome encoding a capsid protein of a virus known to those skilled in the art. Examples of these capsid proteins are AAV capsid proteins VP1, VP2 and VP3. The Cap gene used in the present invention may be derived from any AAV serum type, but is preferably derived from AAV2 or AAV5.

As the vector containing the Rep gene and the Cap gene, for example, a plasmid, a nucleic acid sequence derived from a virus, or an artificially designed nucleic acid can be used, and a plasmid is preferable.

In the present invention, a gene for treatment or prevention may be introduced into cells.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of infectious diseases, inflammatory diseases, autoimmunity, chronic and contagious diseases (including disorders such as acquired immune deficiency syndrome (AIDS), cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation start site (AUG codon) of mRNA) that are useful in antisense therapy against cancer and viral disease.

The gene for therapy or prophylaxis may be linked to a promoter for expressing the gene for treatment or prevention. The promoter for expressing a gene for treatment or prevention is not particularly limited, but examples thereof can include a cytomegalovirus-derived promoter (including an enhancer as desired), an SV40 early promoter, a human elongation factor-1$\alpha$ (EF-1$\alpha$) promoter, a human ubiquitin C promoter, a retrovirus Rous sarcoma virus LTR promoter, a dihydrofolate reductase promoter, a $\beta$-actin promoter, a phosphoglycerate kinase (PGK) promoter, and the like. The gene for treatment or prevention and the promoter for expressing the gene are preferably flanked by the ITR sequences.

As the vector containing a gene for treatment or prevention, for example, a plasmid, a virus-derived sequence, an artificially designed nucleic acid, or the like can be used, and a plasmid is preferable.

In the present invention, a virus helper gene derived from an adenovirus may be preferably introduced into cells. The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

Examples of the virus helper gene derived from adenovirus can include E1A, E1B, E2A, E4, and VA-RNA. In the host cell having all or a part of the E1 region, the region of the adenovirus genome necessary for replicating the AAV genome and packaging of the AAV genome into the capsid to form a AAV virion is the E2A region, the E4 region, and the VA-RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene, or a VA-RNA gene. The VA-RNA gene is preferably a VA-RNA I gene.

The adenovirus-derived virus helper gene (such as an E1A, an E1B, an E2A, an E4, and a VA-RNA) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base are made to the wild-type virus helper gene, the number of the modified bases is preferably 1 to 20, more preferably 1 to 10, and still more preferably 1 to 3. The modified base sequence of the virus helper gene exhibits preferably 85% or more sequence identity, more preferably 90% or more sequence identity, still more preferably 95% or more sequence identity, and even still more preferably 98% or more sequence identity with the base sequence of the wild-type virus helper gene.

In a case of introducing a virus helper gene into a cell, a vector containing the virus helper gene can be introduced into the cell.

As the vector containing a virus helper gene, for example, a plasmid, a virus-derived sequence, an artificially designed nucleic acid, or the like can be used, and a plasmid is preferable.

It is preferable that the virus helper gene is under the control of a promoter.

The specific examples of the promoter are not particularly limited, but can include a cytomegalovirus-derived promoter (CMV promoter) (including an enhancer as desired), an SV40 early promoter, a human elongation factor-1$\alpha$ (EF-1$\alpha$) promoter, a human ubiquitin C promoter, a retrovirus Rous sarcoma virus (RSV) LTR promoter, a dihydrofolate reductase promoter, a $\beta$-actin promoter, and a phosphoglycerate kinase (PGK) promoter. The promoter may be under the control of a promoter capable of regulating expression. The promoter capable of regulating expression may be a Tet-on/off system which is a promoter capable of regulating expression by tetracycline, or a Tet-on system.

The nucleic acid used in the present invention preferably contains four or more genes selected from the group consisting of a gene for treatment or prevention, a Rep gene, a Cap gene, an E2 gene, an E4 gene, and a VA-RNA1 gene.

In the present invention, the nucleic acid is preferably introduced into the cell using one or more plasmids.

The nucleic acid is preferably a plasmid containing a gene for treatment or prevention, a plasmid containing a Rep gene and a Cap gene, and a plasmid containing an E2 gene, an E4 gene, and a VA-RNA1 gene.

The nucleic acid may include a plasmid containing a Rep gene, an E2 gene, an E4 gene, and a VA-RNA1 gene, and a plasmid containing a Cap gene and a gene for treatment or prevention.

In the present invention, the nucleic acid can be introduced into the cell using a plurality of plasmids. Provided that the form of the introduced nucleic acid is not limited as long as the purpose of introducing the nucleic acid encoding the gene necessary for virus production into the cell is achieved. For example, a large plasmid in which the above-described gene is carried in one plasmid may be used. Provided that since in electroporation, as the size of the plasmid increases, the cell damage during electroporation tends to increase, the size of the plasmid is preferably 15 kbp or less.

The method for producing a virus may be any of a triple transfection (TT) method or a packaging cell method.

The TT method is a method in which a plasmid containing a Rep gene and a Cap gene, an adenovirus helper plasmid, and a plasmid containing a transgene (for example, a gene for desired therapy or prophylaxis) are simultaneously transfected into cells, and can thus be packaged as recombinant AAV (rAAV).

The packaging cell method is a method of producing AAV by introducing, to the cell in which a part of the gene in the TT method is incorporated into a chromosome in advance, the remaining genes into a cell by transfection.

The method for producing a virus according to the embodiment of the present invention includes a culture step of culturing the cells into which the nucleic acid has been introduced.

The culture of cells can be performed under normal conditions for cell culture.

The culture temperature of the cells is not particularly limited, and the culture of the cells are performed at a temperature at which the cells can survive. The culture temperature is generally 25°C to 45°C, preferably 30°C to 42°C, more preferably 35°C to 40°C, and 37°C as an example.

The $CO_2$ concentration is generally 3 to 10% $CO_2$, preferably 5 to 10% $CO_2$, and 8% $CO_2$ as an example.

As the culture conditions, the suitable conditions in the preculture may be applied as they are. It is noted that, in a case of performing electroporation, since there are effects of perforation damage due to electroporation, expression of introduced genes, immune response due to virus accumulation in cells, toxicity, and the like, the culture conditions may be optimized to have lower damage than the preculture (proliferation culture).

The cell density is preferably equal to or less than that in the preculture. The cell density is preferably $1 \times 10^6$ to $10 \times 10^6$ cells/mL in a case of the batch culture, and preferably $1 \times 10^6$ to $20 \times 10^6$ cells/mL in a case of the fed-batch culture.

The cell concentration may be diluted in the subsequent culture step after the gene introduction.

The period of the virus production is preferably 24 hours or more and 90 days or less, more preferably 24 hours or more and 30 days or less, still more preferably 24 hours or more and 20 days or less, even still more preferably 24 hours or more and 10 days or less, yet even still more preferably 24 hours or more and 7 days or less, and particularly preferably 24 hours or more and 72 hours or less. Since electroporation does not have an intracellular introduction, endosome escape, and a PEI slow release process in principle unlike the PEI method, the nuclear reaching to expression speed of the introduced gene is faster than that of the PEI method. In the typical PEI method, the virus production period is 72 hours, whereas in the EP, it is preferably about 48 hours.

As the culture, batch culture, fed-batch culture, perfusion culture, or shaking culture can be performed. From the viewpoint of large-scale processing and high cell density, it is preferable that at least a part of the virus production step is fed-batch culture (fed-batch culture) or perfusion culture.

In the culture step of culturing the cells into which the nucleic acid has been introduced, fed-batch culture or perfusion culture is preferable. In a case where the culture step of culturing the cells into which the nucleic acid has been introduced is performed by perfusion culture, in a case where the nucleic acid is introduced into the cells using the gene introduction reagent, it is preferable that the perfusion culture is not started for 4 to 24 hours immediately after the nucleic acid introduction step, and subsequently the perfusion culture is performed. The perfusion ratio in this case is preferably 0.5 or more and more preferably 1 or more. The perfusion ratio is still more preferably 1 to 5. The perfusion ratio may be 2 to 5 or 3 to 5. By not performing the perfusion culture for 4 to 24 hours immediately after the nucleic acid introduction step, the nucleic acid can be held in the culture medium, and there is an advantage that the destruction of the complex of the nucleic acid and the gene introduction reagent can be suppressed. The time during which the perfusion culture is not performed is more preferably 6 hours or more, more preferably 9 hours or more, and more preferably 12 hours or more.

The perfusion culture is a culture method in which a fresh culture medium is supplied into a cell culture solution and a part of the culture medium in which cells are cultured is removed. In a case where this perfusion culture is carried out, it is possible to remove, from a culture tank, metabolic decomposition products discharged from cells. In the perfusion culture, it is possible to collect a liquid obtained by continuously separating cells in a culture solution while continuously supplying a culture medium to a culture tank.

In general, the perfusion culture makes it possible to achieve a high viable cell density. A typical perfusion culture starts with a batch culture lasting one or two days, subsequently, a fresh feed medium is continuously, in phases, and/or intermittently added to the culture product, and the used medium is removed at the same time. In the perfusion culture, it is also possible to separate cells by using methods such as sedimentation, centrifugation, and filtration and remove the used culture medium while maintaining the viable cell density. The advantage of the perfusion culture is that the cell proliferation rate in a case of increasing the cell density is maintained for a longer period than in the batch culture method or the fed-batch culture.

Perfusion may be in any form of continuous perfusion, phased perfusion, intermittent perfusion, or a combination thereof. A continuous form is preferable. The cells are retained in the culture product and the used medium that is removed may substantially do not include cells or may have much fewer cells than the culture product. The virus expressed by a cell culture can be retained in the culture product or collected by selecting the membrane pore diameter.

In an example of the present invention, the preculture step of culturing the suspension containing cells having a density of $10 \times 10^6$ cells/mL or more is performed, and in a case where the population cell doubling time of the suspension satisfies 35.4 hours or less, a part of the culture product of the preculture can be collected, a nucleic acid is added to a part of the collected culture product, and the cells into which the nucleic acid has been introduced can be cultured to produce a virus. Then, the culture of an uncollected remaining part of the culture product of the pre-culture is continued, and subsequently, in a case where a population cell doubling time of the suspension satisfies 35.4 hours or less, a part of the culture product can be collected and viral production by nucleic acid introduction and cell culture can be performed. From the viewpoint of maintaining the cell density of the suspension at a predetermined value, it is preferable to continue the culture of the remaining part, to collect a part of the culture product in a case where a population cell doubling time of the suspension satisfies 35.4 hours or less at least 4 hours after the culture, and to perform viral production by nucleic acid introduction and cell culture.

The continuous separation method for a cell culture solution in the perfusion culture is preferably a method performing continuous separation using a membrane, and is preferably membrane filtration. The membrane filtration for separating cells and a product in the culture solution is more preferably alternating tangential flow filtration (ATF method).

Devices and culture methods for perfusion culture are known in the related art, and are described in WO2018/159847, WO2019/049843A, WO2019/181234A, WO2019/239780A, WO2020/003833A, WO2020/162125A, WO2021/187008A, WO2022/196710A, and WO2023/054556A, the contents of which are incorporated herein by reference.

A method for collecting a virus from a culture solution can be performed by draining from the secondary side of the filtration membrane with a pump, but another available liquid feeding means may be used. The extracted culture solution is subjected to treatments such as collection of the virus and removal of the dead cells. In addition, the extracted culture solution may be partially discarded or returned to the culture container after treatments such as collection of the virus and removal of the dead cells. In a case where a loss of the culture solution occurs due to the above treatments, the loss can be compensated, for example, by supplying a fresh culture medium to the culture container.

In the perfusion culture, it is possible to adjust a cell density to be within $\pm$ 10% of the target cell density by collecting a culture solution containing cells at least once or more a day after the cell density reaches a target cell density.

Extracting a part of a culture solution together with cells so that the viable cell density during the culturing does not become excessive, thereby reducing the viable cell density is referred to as cell bleeding, and the volume of the culture solution can be maintained by adding the same volume of a fresh culture medium as the amount of the extracted culture solution. The term "once or more a day" includes a case of continuously and automatically carrying out cell bleeding.

The culture container is not particularly limited, and may be, for example, any one of a flask or a bioreactor. The capacity of the culture container is preferably 1 L or more, more preferably 10 L or more, more preferably 50 L, more preferably 100 L or more, and more preferably 500 L. The upper limit is 200,000 L.

The culture scale is not particularly limited, and the cells can be cultured in a culture medium of any volume, for example, the cells can be cultured in a culture medium of 1 mL to 2,500 L, preferably 1 L to 2,300 L, more preferably 50 L to 2,200 L, and particularly preferably 300 L to 2,100 L.

In cell culture, a gas containing oxygen can be introduced into a culture solution using a sparger. It is preferable that the dissolved oxygen concentration of the culture solution is adjusted by introducing a gas containing oxygen into the culture solution. The dissolved oxygen concentration in the culture solution can be appropriately set and is not particularly limited; however, it is 10% to 150%, preferably 15% to 120%, and more preferably 20% to 100% in a case where the saturated dissolved oxygen concentration in the liquid at 37°C in air at 1 atm is set to 100%.

The pore diameter of the sparger is not particularly limited, but is preferably 5 $\mu$m to 100 $\mu$m, and more preferably 10 $\mu$m to 50 $\mu$m.

The ventilation amount of the gas containing oxygen is not particularly limited, but is generally 0.001 to 1.0 vvm and preferably 0.005 to 0.5 vvm. VVM is volume per volume per minute.

The culture may be performed while stirring by shaking. The stirring speed in the case of stirring by shaking is generally 50 rpm to 200 rpm, and preferably 80 to 150 rpm.

The stirring culture may be rotary stirring culture using an impeller, a propeller, a paddle, or the like in a reactor. For example, a three-blade propeller, a two-blade paddle, or the like can be used. The size of the impeller, the propeller, or the paddle is set according to the size of the culture tank.

The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

In the culture, a membrane separation treatment step of passing a cell suspension extracted from a culture tank through a separation membrane to separate the cell suspension into a cell-containing liquid and a permeated liquid may be performed. In this operation, the cell suspension extracted from the culture tank is separated into a cell-containing liquid having a cell concentration higher than that of the cell suspension and a permeated liquid having a cell concentration lower than that of the cell suspension.

The membrane separation treatment step is preferably tangential filtration, more preferably alternating tangential flow (ATF) or tangential flow, and most preferably ATF. Examples of the filter that can perform ATF include SuUATF10-S02PES, F2 RF02PES, or the like, manufactured by Repligen Corporation.

Examples of the material of the membrane used in the membrane separation treatment step include polyethersulfone, modified polyethersulfone, mixed cellulose ester, and the like.

The pore diameter of the membrane used in the membrane separation treatment step is preferably 0.1 $\mu$m to 0.4 $\mu$m and more preferably 0.15 $\mu$m to 0.3 $\mu$m.

In the present invention, it is preferable that the cells are in a suspension in the nucleic acid introduction step and the culture step, and the suspension is a culture medium.

In the culture according to the embodiment of the present invention, which includes the preculture, cell bleeding may be performed by withdrawing a part of the culture solution. By performing the cell bleeding, the cell density can be maintained at a predetermined value. The cell bleeding can be performed by the following method, as an example. After measuring the cell concentration, in a case of withdrawing the culture solution, the pump is operated at a rate of 0.3 vvd to 3 vvd. The cell liquid obtained by cell bleeding may be transferred to a culture tank separate from the preculture. The cell bleeding is performed once or more a day, and the cell bleeding performed last in a day is preferably performed within 5 hours and more preferably within 3 hours from the cell bleeding performed first. After the cell bleeding is completed in a day, a volume of cell culture medium equal to the amount of cell suspension reduced by the cell bleeding is added, thereby returning the total culture volume in the preculture to the level before the cell bleeding, and the culture is continued. The cell bleeding can also be performed by automatic control. In a case where the cell bleeding was automatically controlled, the cell bleeding can be continuously performed automatically, for example, using a FUTURA sensor manufactured by ABER Instruments Ltd. while monitoring the electrostatic capacity of the culture solution. As an example, the method can be performed by the following method.

(1) An electrostatic capacity serving as a target is set.
(2) A weight control value of the culture tank is set.
(3) The electrostatic capacity of the culture solution is measured at a period of 0.1 s or less.
(4) A pump for withdrawing the culture solution is driven at a rate of 0.3 vvd to 3 vvd in a case where the electrostatic capacity of the culture solution exceeds the target.
(5) A culture medium is automatically supplied into the culture tank while the weight of the culture tank is measured, to keep the liquid volume of the culture solution approximately constant.
(6) The pump for withdrawing the culture solution is stopped in a case where the electrostatic capacity of the culture solution is smaller than the target by 0.01% or more.

The cell bleeding rate is preferably 10% to 50%/day and more preferably 20% to 50%/day. The cell bleeding rate is a proportion of the culture solution withdrawn per day with respect to the total volume of the culture solution.

Examples of the culture medium include, but are not limited to, a BalanCD (registered trademark) culture medium, Expi293 Expression Medium (Thermo Fisher Scientific, A1435101), a Dulbecco's modified Eagle's medium (DMEM) containing 10% (vol/vol) fetal bovine serum (FBS), Gibco (trademark) Viral Production Medium (Thermo Fisher Scientific, A4817901), a serum-free UltraCULTURE (trademark) culture medium (Lonza), a HuMEC basal serum-free culture medium, a KNOCKOUT (trademark) CTS (trademark) XenoFREE ESC/iPSC culture medium, a STEMPRO (trademark)-34 SFM culture medium, a STEMPRO (trademark) NSC culture medium, an ESSENTIAL (trademark)-8 culture medium, a culture medium 254, a culture medium 106, a culture medium 131, a culture medium 154, a culture medium 171, a culture medium 200, a culture medium 231, a HepatoZYME-SFM, a human endothelial-SFM, a Gibco (registered trademark) FREESTYLE (trademark) 293 expression culture medium, a culture medium 154CF/PRF, a culture medium 154C, a culture medium 154 CF, a culture medium 106, a culture medium 200PRF, a culture medium 131, an Essential (trademark)-6 culture medium, a STEMPRO (trademark)-34 culture medium, a Gibco (registered trademark) Astrocyte culture medium, an AIM V (registered trademark) CTS (trademark) culture medium, an AMINOMAX (trademark) C-100 basal culture medium, an AMINOMAX (trademark)-II complete culture medium, a CD FORTICHO (trademark) culture medium, a CD CHO AGT culture medium, a CHO-S-SFM culture medium, a Gibco (registered trademark) FREESTYLE (trademark) CHO expression culture medium, a CD OPTICHO (trademark) culture medium, a CD CHO culture medium, a CD DG44 culture medium, a SF-900 (trademark) culture medium, an EXPI293 (trademark) expression culture medium, an LHC basal culture medium, an LHC-8 culture medium, a 293 SFM culture medium, a CD 293 culture medium, an AEM growth culture medium, a PER.C6 (registered trademark) cell culture medium, an AIM V (registered trademark) culture medium, an EXPILIFE (registered trademark) culture medium, a keratinocyte SFM culture medium, an LHC culture medium, an LHC-8 culture medium, an LHC-9 culture medium, and any derivatives or modifications thereof. In a certain specific non-limiting embodiment, the high-density culture medium may be a CD FORTICHO (trademark) culture medium, a CD CHO AGT culture medium, a CHO-S-SFM culture medium, a GIBCO (registered trademark) FREESTYLE (trademark) CHO expression culture medium, a CD OPTICHO (trademark) culture medium, a CD CHO culture medium, a CD DG44 culture medium, a GIBCO (registered trademark) FREESTYLE (trademark) 293 expression culture medium, an EXPI293 (trademark) expression culture medium, an LV-MAX (trademark) production culture medium, a FREESTYLE (trademark) F17 expression culture medium, a DYNAMIS (trademark) culture medium, a similar culture medium, or a modified form thereof. Alternatively, a homemade culture medium may be used. Additional component may be appropriately supplemented to the culture medium as necessary. Examples of the additional component include amino acids, salts, sugars (glucose and the like), vitamins, hormones, growth factors, lipids, trace elements, and the like, but the additional component is not particularly limited. The pH of the culture medium is 6 to 8, preferably 6.8 to 7.6, and more preferably 7.2 to 7.6. The culture medium may be supplemented with 1 × GlutaMAX (TM) (manufactured by Thermo Fisher Scientific, Inc.) or the like, as desired. Among these, a BalanCD (registered trademark) culture medium, Expi293 Expression Medium (Thermo Fisher Scientific, A1435101), a Dulbecco's modified Eagle's medium (DMEM) containing 10% (vol/vol) fetal bovine serum (FBS), Gibco (trademark) Viral Production Medium (Thermo Fisher Scientific, A4817901), or FREESTYLE (trademark) F17 expression culture medium is particularly preferable. These culture media contain glucose and amino acids necessary for the proliferation of HEK cells. By performing the perfusion culture using these culture media, it is important for virus production from high-density cells, from the viewpoint of increasing the proliferative activity of the cells, to continuously supply nutrients necessary for the proliferation of HEK cells while washing away proliferation-inhibiting components such as ammonia and lactic acid secreted into the culture medium, to maintain a cell environment in which these components are in a low concentration.

An anti-foaming agent may be further added to the culture medium. As the anti-foaming agent, a silicone-based anti-foaming agent is preferable, and dimethicone is particularly preferable. The anti-foaming agent is preferably an anti-foaming component containing polydimethylsiloxane, and it is more preferably an anti-foaming component (simethicone) in which fine powder silica is contained in polydimethylsiloxane. The addition rate of the simethicone with respect to the volume of the culture solution is not particularly limited, but is preferably 5 mg/hr/L or less and more preferably 2 mg/hr/L or less.

A block copolymer of polyoxypropylene and polyoxyethylene may be added to the culture medium. The copolymer of polyoxypropylene and polyoxyethylene is preferably Poloxamer, where Poloxamer-188 is more preferable. The content of the block copolymer of polyoxypropylene and polyoxyethylene in the culture medium is not particularly limited; however, it is preferably 0.1% by mass or more and 2% by mass or less, more preferably 0.25% by mass or more and 2% by mass or less, and still more preferably 0.5% by mass or more and 2% by mass or less.

In the present invention, preferably, the produced virus can be collected.

The virus particles produced in the cell are present in the culture solution or in the cell (in the nucleus). The extraction from the inside of the cell (destruction/dissolution of the cell membrane and the nuclear membrane) may be performed by a freeze-thawing method, or may be performed by adding a surfactant (such as Triton X) and stirring. The separation of the virus and the cell debris can be performed by centrifugation or depth filtration.

The virus may be purified by performing TFF (concentration/buffer exchange), affinity chromatography, or AEX (anion exchange chromatography) treatment as necessary. In this manner, it is possible to remove cell debris, cell-derived nucleic acids, and cell-derived proteins. Alternatively, a commercially available virus purification kit (such as an AAV purification kit) may be used. For example, an AAVpro (registered trademark) Purification Kit Maxi/Midi manufactured by Takara Bio Inc. or the like can be used.

The titer of the virus produced in the present invention can be measured by a conventional method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding $MgCl_2$ and Benzonase to the collected supernatant to react. It is possible to measure the titer of a target virus by performing droplet digital PCR (ddPCR) using the sample containing the target virus obtained above as a ddPCR sample.

Furthermore, a proportion of capsids containing a GOI (Full ratio) can be calculated from the ratio of the capsid particle titer (Vp/mL) measured using the ELISA kit to the genome titer (vg/mL) calculated by ddPCR measurement. Alternatively, the proportion of capsids containing a GOI (Full ratio) may be obtained by analyzing the sample solution after virus extraction and purification by HPLC measurement, capillary isoelectric focusing, or the like in the same measurement system.

In the produced virus according to the embodiment of the present invention, the proportion of the capsids containing a GOI (Full ratio) is preferably 5% or more, more preferably 7% or more, still more preferably 10% or more, even still more preferably 20% or more, yet even still more preferably 30% or more, and particularly preferably 50% or more.

According to the present invention, there is provided a method for introducing a nucleic acid into cells, the method including a nucleic acid introduction step of adding a nucleic acid to a suspension containing cells having a density of $10 \times 10^6$ cells/mL or more, in which the nucleic acid is added in a case where a population cell doubling time of the suspension satisfies 35.4 hours or less.

According to the present invention, furthermore, there is provided a method for introducing a nucleic acid into cells, the method including a nucleic acid introduction step of adding a nucleic acid to a suspension containing cells having a density of $10 \times 10^6$ cells/mL or more, in which the nucleic acid is added in a case where a population cell doubling time of the suspension satisfies 35.4 hours or less.

Preferred aspects of the method for introducing a nucleic acid into cells according to the embodiment of the present invention are as described above in the present specification.

The present invention will be more specifically described using the following examples; however, it is not limited by the examples.

Examples

<Example 1>

[Cell culture]

HEK293 cells (Expi293, A14527, manufactured by Thermo Fisher Scientific, Inc.) were cultured in a BalanCD medium (manufactured by Fujifilm Irvine Scientific, Inc.) supplemented with 1×GlutaMAX (TM) (manufactured by Thermo Fisher Scientific, Inc.). The cells were cultured in a shaking flask (manufactured by Corning Inc.) or a bioreactor. In the shaking flask, the cells were cultured in a 37°C incubator with stirring at 120 rpm in a humidified atmosphere of 8% $CO_2$.

[Batch culture]

The cell culture by a batch culture mode were performed using the above-described HEK293 cells. As the culture medium, BalanCD culture medium (manufactured by Fujifilm Irvine Scientific, Inc.) was used, and the seeding density of the cells was set to $5 \times 10^5$ cells/mL. The culture environment was maintained so that the pH was 6.4 or more and 7.6 or less, the culture temperature was 36°C or more and 38°C or less, and the $CO_2$ concentration was 25% or less.

After the start of the culture, on the second day on which the cell density reached $2 \times 10^6$ cells/mL, the plasmid DNA was introduced using the PEI reagent which will be described later.

The cell density was measured at 9:00 a.m., and the nucleic acid was added within 1 hour after the measurement.

[Fed-batch culture]

The cell culture by a fed-batch culture mode was performed using the above-described HEK293 cells. As the culture medium, BalanCD culture medium (manufactured by Fujifilm Irvine Scientific, Inc.) was used, and the seeding density of the cells was set to $5 \times 10^5$ cells/mL. The culture environment was maintained so that the pH was 6.4 or more and 7.6 or less, the culture temperature was 36°C or more and 38°C or less, and the $CO_2$ concentration was 25% or less.

After the start of the culture, on the second day or later on which the cell density reached $2 \times 10^6$ cells/mL, BalanCD Feed culture medium (manufactured by Fujifilm Irvine Scientific, Inc.) was added once a day such that the glucose concentration in the culture medium was 4 g/L and the culture was continued. After the start of the culture, on the seventh day on which the cell density reached $20 \times 10^6$ cells/mL, the plasmid DNA was introduced using the PEI reagent which will be described later.

The cell density was measured at 9:00 a.m., and the nucleic acid was added within 1 hour after the measurement.

[Perfusion culture]

Cell culture by a perfusion culture mode was performed using the above-described HEK293 cells. The perfusion ratio was set to 3. As the culture medium, BalanCD culture medium (manufactured by Fujifilm Irvine Scientific, Inc.) was used, and the seeding density of the cells was set to $5 \times 10^5$ cells/mL. The culture environment was maintained so that the pH was 6.4 or more and 7.6 or less, the culture temperature was 36°C or more and 38°C or less, and the $CO_2$ concentration was 25% or less.

In the culture capacity of 1 L or less, a glass container having a diameter of 114 mm was used as the culture container, a paddle-shaped stirring blade having a diameter of 85 mm was installed, and a predetermined amount of the culture solution was charged into the culture container to carry out culture. For filtration, an RF02PES membrane was used in an ATF2 system manufactured by Repligen Corporation.

On the second day or later after the start of the culture, the perfusion culture including the supply of the fresh culture medium and the removal of the filtered culture solution from the culture container was started. After the start of the culture, on the fifth day on which the cell density reached $20 \times 10^6$ cells/mL, the plasmid DNA was introduced using the PEI reagent which will be described later.

The cell density was measured at 9:00 a.m., and the nucleic acid was added within 1 hour after the measurement.

The perfusion culture was not performed for 4 to 24 hours immediately after the plasmid DNA introduction step (nucleic acid introduction step), and subsequently the perfusion culture was restarted under the condition of the perfusion ratio of 3.

[Gene introduction (nucleic acid introduction)]

As the nucleic acid, the following three plasmid DNAs were used to produce an adeno-associated virus vector (rAAV).

(1) a plasmid containing a gene of interest, which will be described later, with ITRs adjacent to each other,
(2) a packaging plasmid containing a Rep gene and a cap gene, and
(3) an adenovirus helper plasmid containing adenovirus E2, E4, and VA-RNA genes

In all plasmids, AAVpro Packaging Plasmid (AAV5, 6650, manufactured by Takara Bio Inc.) was used. Regarding (1), enhanced green fluorescent protein (EGFP) as the gene of interest was inserted downstream of a cytomegalovirus (CMV)-derived promoter and used.

As the cationic polymer, linear polyethyleneimine (PEI) "PEI pro" having a molecular weight of 25 kDa (manufactured by Polyplus) was used as a gene introduction reagent.

A PEI/DNA complex was prepared at a mass ratio between PEI and plasmid DNA of 2:1, and incubated at room temperature for 15 minutes, and the plasmid DNA/PEI complex was added to the cell culture solution. The total amount of the plasmid DNA used for transfection was 0.5 $\mu$g per 1 million cells.

In this case, the plasmid DNA concentration in the suspension was 1.0 $\mu$g/mL in the case of the batch culture, 5.8 $\mu$g/mL in the case of the fed-batch culture, and 5.6 $\mu$g/mL in the case of the perfusion culture.

[Measurement of cell density and viability]

The cell density and the survival rate were determined using Vi-cell (trademark) XR cell viability analyzer (manufactured by Beckman Coulter).

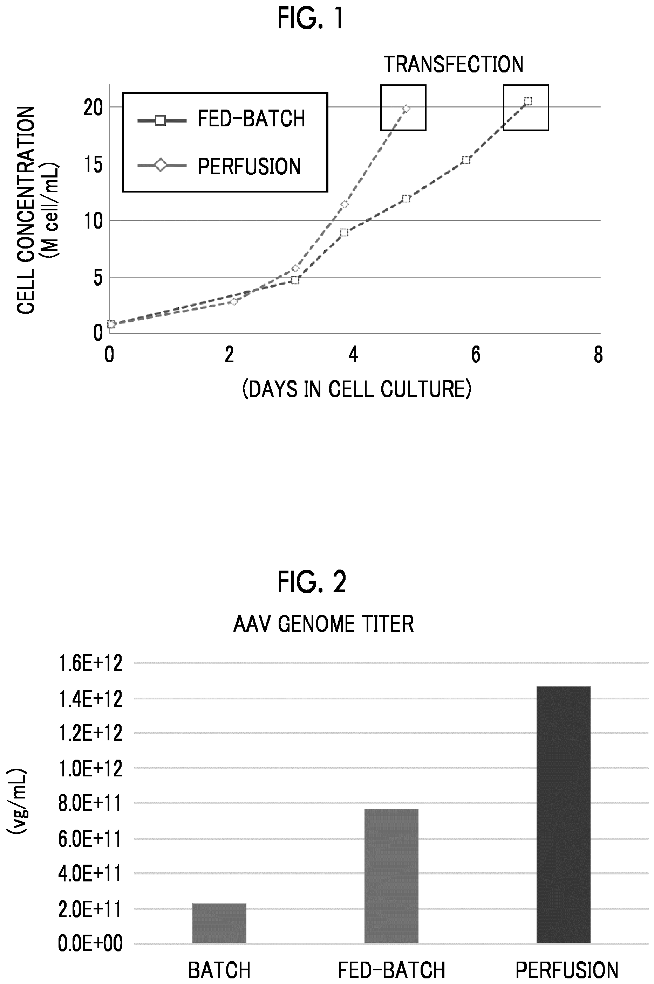

FIG. 1 shows a result of measuring a change in cell density during the culture period in a case of the fed-batch culture and a case of the perfusion culture.

In the fed-batch culture, the cell density on the seventh day was $20.4 \times 10^6$ cells/mL, and the cell density on the sixth day was $15.9 \times 10^6$ cells/mL. That is, a ratio of the cell density A at the time point A to the cell density B at the time point B which is 24 hours before the time point A, that is, the cell density A/cell density B was 1.28. That is, the population cell doubling time was 66.8 hours.

In the perfusion culture, the cell density on the fifth day was $20.0 \times 10^6$ cells/mL, and the cell density on the fourth day was $11.0 \times 10^6$ cells/mL. That is, a ratio of the cell density A at the time point A to the cell density B at the time point B which is 24 hours before the time point A, that is, the cell density A/cell density B was 1.81. That is, the population cell doubling time was 30.5 hours.

[Measurement of AAV titer]

After 72 hours from gene introduction (nucleic acid introduction), an HEK cell culture solution that had been subjected to culture under the conditions of 37°C and 8% $CO_2$ was collected. Thereafter, Tween 20 (manufactured by FUJIFILM Wako Pure Chemical Corporation) at a final concentration of 0.1%, $MgCl_2$ at a final concentration of 2 mmol/L, and 25 U/mL Benzonase (manufactured by Merck) were added thereto, and the mixture was reacted at 37°C for 2 hours to digest the gDNA (genomic DNA) and the residual plasmid. The sample after the reaction was incubated at 95°C for 15 minutes, 70°C for 3 minutes, 40°C for 3 minutes, and 20°C for 3 minutes in this order, to inactivate Benzonase, and the reactant was used as a ddPCR (Droplet Digital PCR) sample. The ddPCR sample was diluted 50-fold with a TE buffer (pH 8.0, containing 0.05% Pluronic F-68 and 10 $\mu$g/mL bovine thymus DNA). 10 $\mu$L of ddPCRtm Supermix for Probes (no dUTP) (manufactured by Bio-Rad Laboratories, Inc.), 2 $\mu$L of diluted ddPCR sample, primers (seq1, seq2, final concentration of 900 nmol/L) targeted to ITR and probe (seq3, final concentration of 250 nmol/L, FAM (fluorescein amide) was added as a fluorescent probe), nuclease free water (Thermo Fisher Scientific, 10977015) was mixed with each other in a tube on ice, the total liquid volume was adjusted to 22 $\mu$L, and ddPCR was performed.

Seq1 GGAACCCCTAGTGATGGAGTT (SEQ ID NO: 1)
Seq2 CGGCCTCAGTGAGCGA (SEQ ID NO: 2)
Seq3 CACTCCCTCTCTGCGCGCTCG (SEQ ID NO: 3)

In ddPCR, droplets were formed from the prepared solution using a droplet forming apparatus, and subsequently incubated at 95°C for 10 minutes, 94°C for 30 seconds, and 55°C for 60 seconds in this order for 40 cycles, and then incubated at 98°C for 10 minutes. Measurement was performed using a droplet reader, and the AAV genomic titer (vg/mL) was calculated. Table 1 and FIG. 2 show the measurement results of the AAV genome titer. It was confirmed that the AAV genome titer was improved in a case where the population cell doubling time of the suspension satisfied 35.4 hours or less.

[Measurement of full ratio]

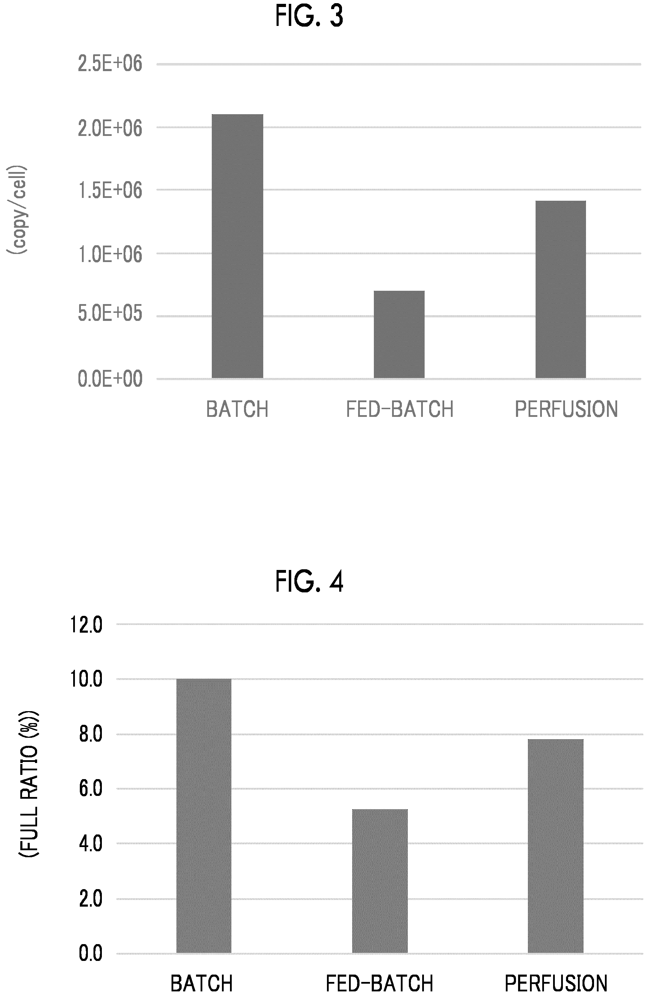

After 72 hours from the gene introduction (nucleic acid introduction), the HEK cell culture solution that had been subjected to culture under the conditions of 37°C and 8% $CO_2$ was collected, and AAV was extracted and purified using AAVpro (registered trademark) Purification Kit manufactured by Takara Bio Inc. The adjustment liquid after purification was analyzed using high performance liquid chromatography to measure the Full ratio (proportion of capsids containing the GOI). An anion exchange column was used as a separation column, and a fluorescence detector (excitation: 280 nm, detection: 348 nm) was used for detection. The Full ratio was calculated from the area value of each of the Empty peak and the Full peak of the AAV. Table 1 and FIG. 4 show the measurement results of the Full ratio. It was confirmed that the Full ratio (proportion of capsids containing the GOI) was improved in a case where the nucleic acid was added in a case where the population cell doubling time of the suspension satisfied 35.4 hours or less (in the case of the perfusion culture) as compared with a case where the population cell doubling time of the suspension did not satisfy 35.4 hours or less (in the case of the fed-batch culture).

[Table 1]

| | Cell density in nucleic acid introduction step (cells/mL) | Doubling time (hour) | AAV genome titer (Vg/mL) | Copy number of gene per cell | AAV genome titer (relative value to batch culture) | Full ratio (%) |
|---|---|---|---|---|---|---|
| Batch culture (Comparative Example) | $2.0 \times 10^6$ | 24.0 | 2.3E+11 | 2.1E+06 | -(1.0) | 9.0 |
| Fed-batch culture (Comparative Example) | $20.4 \times 10^6$ | 66.8 | 7.7E+11 | 7.0E+05 | 3.5 | 4.9 |
| Perfusion culture | $20.0 \times 10^6$ | 30.5 | 1.46E+12 | 1.4E+06 | 5.6 | 7.8 |

[Measurement of copy number of gene per cell]

The copy number of the gene per cell was also evaluated by ddPCR using the ITR sequence associated with the gene of interest as a target, as in the above-described [Measurement of AAV titer]. In this case, since not only the gene of interest encapsulated in AAV but also the gene of interest that was replicated but not encapsulated in AAV were measured together, the following pretreatment of sample was performed. The cells after 72 hours from the gene introduction (nucleic acid introduction) were collected, and Proteinase K (Thermo Fisher Scientific, 25530-049) was added at a final concentration of 0.5 mg/mL to a TE buffer containing SDS at a final concentration of 0.5%, and the collected cell suspension was adjusted to a 15-fold dilution density. The adjustment liquid was subjected to incubation treatment at 55°C for 60 minutes and at 95°C for 10 minutes.

Table 1 and FIG. 3 show the measurement results of the copy number of the gene of interest. It was confirmed that the copy number of the gene of interest was improved in a case where the population cell doubling time of the suspension satisfied 35.4 hours or less (in the case of the perfusion culture).

<Example 2>

[Cell culture]

The culture of HEK293 cells (Viral Production Cells 2.0, A497840, manufactured by Thermo Fisher Scientific) was performed in the same manner as in Example 1.

[Batch culture]

The batch culture was performed using the above-described HEK293 cells in the same manner as in Example 1.

[Fed-batch culture]

The fed-batch culture was performed using the above-described HEK293 cells in the same manner as in Example 1.

[Perfusion culture]

The perfusion culture was performed using the above-described HEK293 cells in the same manner as in Example 1.

[Gene introduction (nucleic acid introduction)]

The gene introduction was performed in the same manner as in Example 1.

[Measurement of cell density and viability]

The cell density and the viability were determined by the method described in Example 1.
In the fed-batch culture, the cell density on the transfection implementation day (culture seventh day) was $20.4 \times 10^6$ cells/mL, and the cell density on the previous day (culture sixth day) was $14.4 \times 10^6$ cells/mL. That is, a ratio of the cell density A at the time point A to the cell density B at the time point B which is 24 hours before the time point A, that is, the cell density A/cell density B was 1.41. That is, the population cell doubling time was 47.8 hours.
In the perfusion culture, the cell density on the transfection implementation day (culture 5th day) was $21.5 \times 10^6$ cells/mL, and the cell density on the previous day (culture 4th day) was $12.0 \times 10^6$ cells/mL. That is, a ratio of the cell density A at the time point A to the cell density B at the time point B which is 24 hours before the time point A, that is, the cell density A/cell density B was 1.79. That is, the population cell doubling time was 28.5 hours.

[Measurement of AAV titer]

The AAV genome titer (vg/mL) was calculated by the method described in Example 1. Table 2 shows the measurement results of the AAV genome titer as a relative value in which the genome titer in the batch culture was set to 1.0. It was confirmed that the AAV genome titer was improved in a case where the nucleic acid was added in a case where the population cell doubling time of the suspension satisfied 35.4 hours or less (in the case of the perfusion culture).

[Measurement of full ratio]

The Full ratio was calculated by performing in the same method as in Example 1. Table 2 shows the measurement results of the Full ratio. It was confirmed that the Full ratio (proportion of capsids containing the GOI) was improved in a case where the nucleic acid was added in a case where the population cell doubling time of the suspension satisfied 35.4 hours or less (in the case of the perfusion culture) as compared with a case where the population cell doubling time of the suspension did not satisfy 35.4 hours or less.
In the perfusion culture, a high Full ratio was obtained even in a case where the fed-batch culture was performed after the gene introduction (nucleic acid introduction).

[Table 2]

|  | Cell density in nucleic acid introduction step (cells/mL) | Doubling time (hour) | AAV genome titer (relative value to batch culture) | Full ratio (%) |
|---|---|---|---|---|
| Fed-batch culture | $20.4 \times 10^6$ | 47.8 | 4.8 | 5.1 |
| Perfusion culture | $21.5 \times 10^6$ | 28.5 | 8.0 | 8.1 |

<Example 3>

[Cell culture]

The cell culture was performed in the same manner as in Example 2.

[Batch culture]

The batch culture was performed in the same manner as in Example 2.

[Continuous-mode culture]

The cell culture was performed by a culture step 1 (preculture) of performing the perfusion culture to increase the density of the cells before the nucleic acid introduction step and a culture step 2 (subsequent culture) of culturing the cells into which the gene was introduced after the nucleic acid introduction step, using the HEK293 cells in the above-described [cell culture]. A separate bioreactor was prepared for each step.

In the culture capacity of 1 L or less, a glass container having a diameter of 114 mm was used as the culture container, a paddle-shaped stirring blade having a diameter of 85 mm was installed, and a predetermined amount of the culture solution was charged into the culture container to carry out culture.

[Continuous-mode culture step 1: preculture step before nucleic acid introduction step]

Cell culture by a perfusion culture mode was performed using the above-described HEK293 cells. For filtration of cell culture medium, an RF02PES membrane was used in an ATF2 system manufactured by Repligen Corporation. As the culture medium, BalanCD culture medium (manufactured by Fujifilm Irvine Scientific, Inc.) was used, and the perfusion ratio was set to 3. The seeding density of the cells was set to $5 \times 10^5$ cells/mL. The culture environment was maintained so that the pH was 6.4 or more and 7.6 or less, the culture temperature was 36°C or more and 38°C or less, and the $CO_2$ concentration was 25% or less.

In the culture step 1, the cell bleeding of drawing out the cell culture solution was performed at a stage where the cell density reached $20 \times 10^6$ cells/mL after the start of the perfusion culture. In the cell bleeding, 40% to 50% of the total volume of the culture in the culture step 1 was drown and the cell culture solution was transferred to a bioreactor for the culture step 2, separate from the culture step 1, in a sterile manner. In a case of performing the cell bleeding of the cell culture solution, the pump was operated at a speed of 0.3 vvd to 3 vvd.

The same volume of BalanCD culture medium (manufactured by Fujifilm Irvine Scientific, Inc.) as the volume of the drawn-out cell culture solution was replenished in the bioreactor of the culture step 1 after the cell bleeding within 1 hour after the cell bleeding.

As a result, the cell density in the bioreactor of the culture step 1 was reduced to about 50% to 60% of that before the cell bleeding.

In the bioreactor of the culture step 1, the perfusion culture was continued, and after 24 hours from the time point at which the cell density was reduced by the cell bleeding, the cell density reached $20 \times 10^6$ cells/mL again.

In the culture step 1, the cell bleeding after the cell density reached $20 \times 10^6$ cells/mL for the first time, the replenishment of the cell culture medium, and the above-described perfusion culture were performed once a day for a total of 22 days of continuous culture.

[Continuous-mode culture step 2: step of culturing cells into which nucleic acid has been introduced after nucleic acid introduction step]

The plasmid DNA was introduced into the cell culture solution of $20 \times 10^6$ cells/mL, which was transferred to a separate bioreactor by the cell bleeding from the culture step 1 in a sterile manner, using the PEI reagent.

The cell density was measured at 9:00 a.m. at the stage of the culture step 1, and the cell bleeding and the addition of the nucleic acid were performed within 1 hour after the measurement.

The plasmid DNA introduction step (nucleic acid introduction step) and the culture step 2 were performed for continuous production Days 1, 8, 15, and 22 in a case where the time point at which the cell density reached $20 \times 10^6$ cells/mL for the first time in the culture step 1 was defined as the continuous production Day 1.

At the time point of the other continuous production Days, the nucleic acid introduction step and the culture step 2 were not performed, and only the culture step 1 (cell bleeding, replenishment of the cell culture medium, and the above-described perfusion culture) was continued.

The culture of the culture step 2 after the nucleic acid introduction step was performed by fed-batch culture under conditions of 37°C and 8% $CO_2$ for 72 hours after the gene introduction.

As the condition of the fed-batch culture, at a time point of 24 or 48 hours after the nucleic acid introduction, a BalanCD Feed Medium (manufactured by Fujifilm Irvine Scientific, Inc.) was added such that the final concentration of the glucose concentration was 6 g/L with respect to the measured glucose concentration, and a glutamine solution (FUJIFILM Wako Pure Chemical Corporation) was added such that the final concentration of the glutamine concentration was 4 mM with respect to the measured glutamine concentration.

[Gene introduction (nucleic acid introduction)]

The gene introduction was performed in the same manner as in Example 1.

[Measurement of cell density and viability]

The cell density and the viability were determined by the method described in Example 1.
The cell density on the day (Day 1, 8, 15, 22) on which the plasmid DNA introduction step was performed was 20.5 x $10^6$ to 24.5 x $10^6$ cells/mL as shown in Table 3. The population cell doubling time T at the time of nucleic acid addition, which was calculated from the cell density at the time point after the cell bleeding on the previous day (Day 0, 7, 14, 21) was 22.8 to 25.2 hours as shown in Table 3.

[Measurement of AAV titer]

The AAV genome titer (vg/mL) was calculated by the method described in Example 1. Table 3 shows the measurement results of the AAV genome titer. It was confirmed that the AAV genome titer was high in all gene introduction batches derived from the continuous production Days in a case where the nucleic acid was added in a case where the population cell doubling time of the suspension satisfied 35.4 hours or less (in the case of the continuous production Days 1, 8, 15, and 22).

[Measurement of full ratio]

The Full ratio was calculated by performing in the same method as in Example 1. Table 3 shows the measurement results of the Full ratio. It was confirmed that the Full ratio (proportion of capsids containing the GOI) was high in gene introduction batches derived from the continuous production Days in a case where the population cell doubling time of the suspension satisfied 35.4 hours or less (in the case of the continuous production Days 1, 8, 15, and 22).

[Table 3]

| | Cell density in nucleic acid introduction step (cells/mL) | Doubling time (hour) | AAV genome titer (relative value to batch culture) | Full ratio (%) |
|---|---|---|---|---|
| Derived from continuous production Day 1 | $20.5 \times 10^6$ | 25.0 | 8.0 | 10.8 |
| Derived from continuous production Day 8 | $21.6 \times 10^6$ | 23.1 | 6.7 | 10.3 |
| Derived from continuous production Day 15 | $24.5 \times 10^6$ | 22.8 | 9.3 | 10.0 |
| Derived from continuous production Day 22 | $24.0 \times 10^6$ | 25.2 | 7.6 | 9.7 |

<Example 4>

[Cell culture]

The cell culture was performed in the same manner as in Example 2.

[Perfusion culture]

Cell culture by a perfusion culture mode was performed using the HEK293 cells of the above-described "Cell culture". The perfusion ratio, the culture medium, the cell seeding density, the culture environment, the culture capacity, the culture container, and the filtration of the perfusion culture were performed in the same manner as in Example 1.
On the second day or later after the start of the culture, the perfusion culture including the supply of the fresh culture medium and the removal of the filtered culture solution from the culture container was started. After the start of the culture, on the seventh day on which the cell density reached $40 \times 10^6$ cells/mL, the plasmid DNA was introduced using the electroporation which will be described later.

The cell density was measured at 9:00 a.m., and the nucleic acid was added and the electroporation was performed within 1 hour after the measurement.

After the electroporation, the cell density was diluted to $16 \times 10^6$ cells/mL within 1 hour.

The culture after the nucleic acid introduction step was performed by fed-batch culture under conditions of 37°C and 8% $CO_2$ for 48 hours after the nucleic acid introduction.

As the condition of the fed-batch culture, at a time point of 24 hours after the nucleic acid introduction, a BalanCD Feed Medium (manufactured by Fujifilm Irvine Scientific, Inc.) was added such that the final concentration of the glucose concentration was 6 g/L with respect to the measured glucose concentration, and a glutamine solution (FUJIFILM Wako Pure Chemical Corporation) was added such that the final concentration of the glutamine concentration was 4 mM with respect to the measured glutamine concentration.

[Gene introduction (nucleic acid introduction)]

The nucleic acid was introduced by electroporation.

The cells were concentrated to $120 \times 10^6$ cells/mL immediately before the electroporation, and subsequently the nucleic acid was added thereto to prepare a suspension to which the nucleic acid had been added. The cell concentration was performed within 1 hour, and the high activated state of cell division was maintained. The concentration of the nucleic acid in the suspension was 60 μg/mL. As the nucleic acid, the same plasmid DNA as in Example 1 was used.

The suspension to which the nucleic acid had been added was filled into a 50 mL syringe.

20 mL of the suspension was fed at 86 mL/min to the electroporation device shown in FIGS. 5 and 6, having an inter-electrode gap of 3 mm, a flow channel width of 20 mm, and an electrode length of 20 mm.

A pulse voltage was applied to the EP electrode, with a voltage amplitude of 412.5 V (electric field strength of 1375 V/cm), pulse width of 3.5 ms, and pulse period of 450 ms. Continuous electroporation was performed on cells flowing in the electrode pair such that, on average, one pulse was applied to each cell.

[Measurement of cell density and viability]

The cell density and the viability were determined by the method described in Example 1.

The cell density on the nucleic acid introduction implementation day (culture seventh day) was $42.3 \times 10^6$ cells/mL. The population cell doubling time T calculated from the cell density on the nucleic acid introduction implementation day (culture seventh day) and the previous day (culture sixth day) was 28.5 hours as shown in Table 4.

[Measurement of AAV titer]

The AAV genome titer (vg/mL) was calculated by the method described in Example 1. Table 4 shows the measurement results of the AAV genome titer. It was confirmed that the AAV genome titer was high in a case where the nucleic acid was added in a case where the population cell doubling time satisfied 35.4 hours or less.

[Measurement of full ratio]

The Full ratio was calculated by performing in the same method as in Example 1. Table 4 shows the measurement results of the Full ratio. It was confirmed that the Full ratio (proportion of capsids containing the GOI) was high in a case where the nucleic acid was added in a case where the population cell doubling time satisfied 35.4 hours or less.

The reason why the Full ratio is particularly high in the nucleic acid introduction by electroporation is not clear, but it is estimated that the nuclear arrival to expression rate of the introduced gene is faster than that in the PEI method.

[Table 4]

| | Cell density in nucleic acid introduction step (cells/mL) | Doubling time (hour) | AAV genome titer (relative value to batch culture) | Full ratio (%) |
|---|---|---|---|---|
| Fed-batch culture | $42.3 \times 10^6$ | 28.5 | 7.2 | 29.7 |

Explanation of References

    10: upper electrode holding plate

20: lower electrode holding plate

30: flow channel plate

32: opening portion (flow channel)

41: electrode pair

41a: upper electrode

41b: lower electrode

50: inflow port

52: outflow port

100: electroporation device

a1: front surface portion of upper electrode

a2: rear surface portion of upper electrode

b1: front surface portion of lower electrode

b2: rear surface portion of lower electrode

L: electrode length

W: electrode width (flow channel width)

D: inter-electrode distance (gap)

[Sequence list] International application 23F00850W1JP24030577_5.xml based on International Patent Cooperation Treaty

**Claims**

1.  A method for producing a virus, the method comprising:

    a nucleic acid introduction step of adding a nucleic acid to a suspension containing cells having a density of $10 \times 10^6$ cells/mL or more; and
    a culture step of culturing the cells into which the nucleic acid has been introduced,
    wherein the nucleic acid is added in a case where a population cell doubling time of the suspension satisfies 35.4 hours or less.

2.  The method according to claim 1,
    wherein a time at which the population cell doubling time of the suspension satisfies 35.4 hours or less is a time at which a cell density A at a time point A and a cell density B at a time point B which is 24 hours before the time point A satisfy the cell density A/the cell density B ≥ 1.6.

3.  The method for producing a virus according to claim 1, further comprising:
    performing perfusion culture of the cells before the nucleic acid introduction step.

4.  The method for producing a virus according to claim 3,
    wherein a perfusion ratio at a time point 24 hours before a start of the nucleic acid introduction step is 1 or more.

5.  The method for producing a virus according to claim 1, further comprising:

a preculture step of culturing the suspension containing the cells having a density of $10 \times 10^6$ cells/mL or more before the nucleic acid introduction step.

6. The method for producing a virus according to any one of claims 1 to 5,
wherein, in the nucleic acid introduction step, the nucleic acid is introduced into the cells using a gene introduction reagent or by electroporation.

7. The method for producing a virus according to any one of claims 1 to 5,
wherein a concentration of the nucleic acid in the suspension is 3 μg/mL or more.

8. The method for producing a virus according to claim 6,
wherein the gene introduction reagent is a cationic polymer.

9. The method for producing a virus according to claim 6,
wherein a mass ratio of the nucleic acid to the gene introduction reagent is 1:1 to 1:3.

10. The method for producing a virus according to any one of claims 1 to 5,
wherein the virus is an adeno-associated virus.

11. The method for producing a virus according to claim 10,
wherein the nucleic acid includes one or more selected from an adeno-associated virus gene and a virus helper gene.

12. The method for producing a virus according to claim 10,
wherein the nucleic acid includes at least one or more virus helper genes.

13. The method for producing a virus according to claim 10,
wherein the nucleic acid includes four or more selected from the group consisting of a gene for treatment or prevention, a Rep gene, a Cap gene, an E2 gene, an E4 gene, and a VA-RNA1 gene.

14. The method for producing a virus according to any one of claims 1 to 5,
wherein the cells are animal cells.

15. The method for producing a virus according to any one of claims 1 to 5,
wherein the cells are HEK cells.

16. The method for producing a virus according to any one of claims 1 to 5,
wherein the nucleic acid is introduced into the cells using one or more plasmids.

17. The method for producing a virus according to any one of claims 1 to 5, further comprising:

a preculture step of culturing the suspension containing the cells having a density of $10 \times 10^6$ cells/mL or more before the nucleic acid introduction step,
wherein, in the culture step, perfusion culture is not performed for 4 to 24 hours immediately after the nucleic acid introduction step, and subsequently perfusion culture is performed.

18. The method for producing a virus according to claim 1, further comprising:

a preculture step of culturing the suspension containing the cells having a density of $10 \times 10^6$ cells/mL or more,
wherein, in a case where a population cell doubling time of the suspension satisfies 35.4 hours or less, a part of a culture product of pre-culture is collected, a nucleic acid is added to a part of the collected culture product, and the cells into which the nucleic acid has been introduced are cultured to produce a virus, and
culture of an uncollected remaining part of the culture product of the pre-culture is continued, and subsequently, in a case where a population cell doubling time of the suspension satisfies 35.4 hours or less, a part of the culture product is collected and viral production by nucleic acid introduction and cell culture is performed.

19. A method for introducing a nucleic acid into cells, the method comprising:

a nucleic acid introduction step of adding a nucleic acid to a suspension containing cells having a density of $10 \times$

$10^6$ cells/mL or more,
wherein the nucleic acid is added in a case where a population cell doubling time of the suspension satisfies 35.4 hours or less.

## FIG. 1

## FIG. 2

## FIG. 3

## FIG. 4

# FIG. 5

# FIG. 6

## INTERNATIONAL SEARCH REPORT

| International application No. |
|---|
| **PCT/JP2024/030577** |

**A.    CLASSIFICATION OF SUBJECT MATTER**

*C12N 7/01*(2006.01)i; *C12N 1/00*(2006.01)i; *C12N 5/10*(2006.01)i; *C12N 5/073*(2010.01)i; *C12N 15/63*(2006.01)i; *C12N 15/87*(2006.01)i; *C12N 15/88*(2006.01)i; *C12N 15/864*(2006.01)i
FI:    C12N7/01 ZNA; C12N15/87 Z; C12N15/88 Z; C12N5/073; C12N15/63 Z; C12N1/00 A ZNA; C12N5/10; C12N15/864 100Z

According to International Patent Classification (IPC) or to both national classification and IPC

**B.    FIELDS SEARCHED**

Minimum documentation searched (classification system followed by classification symbols)

C12N7/01; C12N1/00; C12N5/10; C12N5/073; C12N15/63; C12N15/87; C12N15/88; C12N15/864

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| X | JP 2013-519366 A (CRUCELL HOLLAND BV) 30 May 2013 (2013-05-30) claim 1, paragraphs [0028], [0038], [0054], example 1 | 1-3, 5, 14-16, 19 |
| Y | | 1-19 |
| X | CN 112094814 A (CANSINO BIOLOGICS INC.) 18 December 2020 (2020-12-18) claims 6, 10, 11, paragraphs [0038], [0054], example 5 | 1-3, 5, 14-16, 19 |
| Y | | 1-19 |
| Y | JP 2020-533983 A (BRISTOL-MYERS SQUIBB COMPANY) 26 November 2020 (2020-11-26) claims 1, 15, paragraphs [0100], [0101] | 1-19 |
| Y | JP 2020-524498 A (SPARK THERAPEUTICS, INC.) 20 August 2020 (2020-08-20) claims, paragraphs [0115], [0154]-[0157], examples | 6, 8-13 |
| A | | 1-5, 7, 14-19 |

☑ Further documents are listed in the continuation of Box C.    ☑ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "D"    document cited by the applicant in the international application | "X"    document of particular relevance; the claimed invention cannot be considered novel or cannot be considered to involve an inventive step when the document is taken alone |
| "E"    earlier application or patent but published on or after the international filing date | |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **25 October 2024** | **05 November 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**EP 4 772 616 A1**

**C.    DOCUMENTS CONSIDERED TO BE RELEVANT**

| Category* | Citation of document, with indication, where appropriate, of the relevant passages | Relevant to claim No. |
|---|---|---|
| Y | JP 2021-515576 A (IMMUSOFT CORPORATION) 24 June 2021 (2021-06-24) claim 13, examples | 6, 8, 9 |
| A | | 1-5, 7, 10-19 |
| Y | JP 2008-518632 A (INTROGEN THERAPEUTICS INC.) 05 June 2008 (2008-06-05) claims 1-3 | 10-13 |
| A | | 1-9, 14-19 |
| Y | JP 2023-518919 A (CEVEC PHARMACEUTICALS GMBH) 08 May 2023 (2023-05-08) claims | 10-13 |
| A | | 1-9, 14-19 |

Form PCT/ISA/210 (second sheet) (July 2022)

**INTERNATIONAL SEARCH REPORT**

International application No.

**PCT/JP2024/030577**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT
### Information on patent family members

International application No.

**PCT/JP2024/030577**

| Patent document cited in search report | | | Publication date (day/month/year) | Patent family member(s) | | | Publication date (day/month/year) |
|---|---|---|---|---|---|---|---|
| JP | 2013-519366 | A | 30 May 2013 | US | 2012/0315696 | A1 | |
| | | | | claim 13, paragraphs [0032], [0041], [0056], example 1 | | | |
| | | | | WO | 2011/098592 | A1 | |
| CN | 112094814 | A | 18 December 2020 | (Family: none) | | | |
| JP | 2020-533983 | A | 26 November 2020 | US | 2020/0255785 | A1 | |
| | | | | claims 1, 15, paragraphs [0108], [0109] | | | |
| | | | | WO | 2019/055796 | A1 | |
| JP | 2020-524498 | A | 20 August 2020 | US | 2020/0165632 | A1 | |
| | | | | claims, paragraphs [0122], [0161]-[0164], examples | | | |
| | | | | WO | 2018/226887 | A1 | |
| JP | 2021-515576 | A | 24 June 2021 | US | 2021/0047619 | A1 | |
| | | | | claim 13, examples | | | |
| | | | | WO | 2019/178613 | A1 | |
| JP | 2008-518632 | A | 05 June 2008 | US | 2010/0105124 | A1 | |
| | | | | paragraph [0019] | | | |
| | | | | WO | 2006/052302 | A2 | |
| JP | 2023-518919 | A | 08 May 2023 | WO | 2022/112218 | A1 | |
| | | | | claims | | | |
| | | | | EP | 4006141 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020524498 A
- JP 2023518919 A
- WO 2018159847 A
- WO 2019049843 A
- WO 2019181234 A
- WO 2019239780 A
- WO 2020003833 A
- WO 2020162125 A
- WO 2021187008 A
- WO 2022196710 A
- WO 2023054556 A
- WO 2022224803 A
- WO 2023157673 A
- WO 2023223931 A
- WO 2015005430 A
- JP 24030577 B